# FASCICULE DE BREVET EUROPEEN

(11) **EP 1 094 771 B1**
(45) Date de publication et mention de la délivrance du brevet: **19.05.2004**
(21) Numéro de dépôt: 99926582.0
(22) Date de dépôt: 01.07.1999
(51) Int. Cl.: A61F 13/08

(54) **ORTHESE COMPRESSIVE DU TYPE BAS OU COLLANT DE CONTENTION**
KOMPRESIONSSTRUMPF ODER -STRUMPFHOSE
COMPRESSIVE ORTHOSIS SUCH AS RETENTION STOCKING OR TIGHTS

(30) Priorité: 06.07.1998 FR 9808638
(43) Date de publication de la demande: 02.05.2001
(73) Titulaire: Innothera Topic International (Societe Anonyme), 94110 Arcueil (FR)
(72) Inventeur: GARDON-MOLLARD, Christian, F-63400 Chamalières (FR); GUYOT, François, CH-2072 Saint Blaise (CH)
(74) Mandataire: Dupuis-Latour, Dominique
(86) Numéro de dépôt international: PCT/FR1999/001585
(87) Numéro de publication internationale: WO 2000/001332

(56) Documents cités:
- FR-A- 2 654 925
- FR-A- 2 749 754
- US-A- 3 889 494
- US-A- 4 745 917

## Description

L'invention a trait au domaine des orthèses compressives d'un ou des deux membres inférieurs, généralement connues sous la dénomination "bas de contention" ou "collants de contention".

Bien que dans la suite on utilisera les termes "bas" ou "collant", l'invention n'est pas limitée à un article particulier, mais s'applique aussi bien à toutes les orthèses compressives, qu'il s'agisse de collants (couvrant les deux membres inférieurs et l'abdomen jusqu'à la ceinture, en une seule pièce), de mono-collants (collant muni d'une seule jambe, destiné à la contention d'un seul des membres inférieurs), de bas (couvrant la cuisse et le jarret) ou de chaussettes (couvrant le jarret seul).

Pour permettre une compression forte du ou des membres inférieurs, ces articles sont réalisés en un matériau élastique, typiquement une maille tricotée de texture très serrée.

Les FR-A-2 654 925, FR-A-2 749 754, US-A-4 745 917 et US-A-3 889 494 décrivent de telles orthèses compressives à visée thérapeutique.

L'un des inconvénients de cette texture très serrée est son port assez peu confortable, tout particulièrement lorsque la température ambiante est élevée, typiquement en période estivale. Du fait de cet inconfort, générateur de sudation et d'irritations, les patientes ont tendance à abandonner ou réduire le port de ces orthèses dans ces circonstances, alors même qu'une contention continue, même réduite, serait souhaitable sur le plan thérapeutique.

L'invention a pour but de remédier à cette difficulté en proposant un nouveau type d'orthèse compressive qui, tout en procurant l'intégralité de l'effet thérapeutique recherché (contention/compression à un degré thérapeutique, donc avec dégressivité à partir de la cheville), offre une sensation de bas plus frais, plus aéré permettant d'améliorer grandement le confort de la patiente et par voie de conséquence l'observance du traitement.

L'orthèse compressive de l'invention, qui est du type du type bas ou collant de contention comprenant une partie de jambe en maille compressive apte à délivrer une contention thérapeutique dégressive, est caractérisée en ce que ladite maille compressive comprend une maille de type résille tricotée avec un fil de trame en matière élastique.

Très avantageusement, la maille de type résille est combinée à au moins un fil de fond en matière élastique, de plus faible titrage que le fil de trame.

Selon diverses caractéristiques préférentielles :
― la maille comprend au moins deux fils de fond pour un fil de trame, de préférence trois fils de fond pour un fil de trame, le(s) fil(s) de fond étant tricoté(s) avec le fil de trame de manière à former avec celui-ci un réseau de cellules sensiblement hexagonales, notamment un réseau présentant à la déformation une plus grande force de rappel élastique dans la direction horizontale que dans la direction verticale ;
― le ratio du titrage du fil de fond par rapport au titrage du fil de trame est d'au moins 3:1, de préférence environ 4:1 ; le titrage du fil de trame est de préférence d'environ 130 dtex et celui du fil de fond d'environ 33 dtex.

On va maintenant décrire un exemple de réalisation de l'invention, en référence aux dessins annexés.

La figure 1 est une vue générale montrant les différentes parties d'un collant de contention.

La figure 2 montre le détail de la maille tricotée selon les enseignements de l'invention.

La figure 3 est une macrophotographie montrant la texture du bas selon l'invention.

La figure 4 est un agrandissement de la vue de la figure 3.

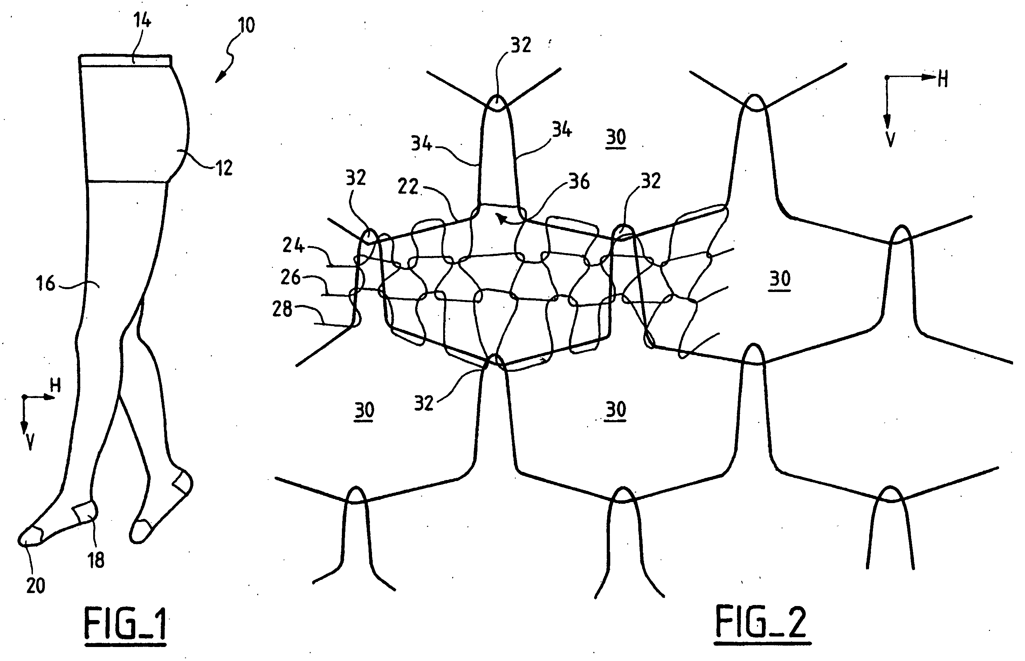

Sur la figure 1, la référence 10 désigne de façon générale un collant de contention, qui comporte, de manière en elle-même connue, une partie de culotte 12, avec une ceinture 14, et une partie de jambe 16 renforcée en 18 et 20 aux talons et aux pointes.

La culotte 12 avec la ceinture 14, ainsi que les renforts de talons 18 et de pointes 20 sont tricotés de manière en elle-même classique, par exemple avec une maille micromesh ou jersey, que l'on ne décrira pas plus en détail.

La partie de jambe 16 est conçue de manière à procurer une contention thérapeutique, typiquement une contention de classe I ou II (classe selon les normes françaises) avec une pression à la cheville de l'ordre de 10 à 15 mm Hg (13,3 à 20,0 hPa) pour la classe I et 15 à 20 mm Hg (20,0 à 26,6 hPa) pour la classe II. En outre cette pression diminue de la cheville à la cuisse, la pression en haut de la jambe étant inférieure à 50 % de la pression au niveau de la cheville ; il s'agit d'obtenir un gradient de pression régulièrement dégressif de la cheville à la cuisse pour faciliter le retour veineux.

La partie de jambe 16 présente un tricotage particulier permettant d'obtenir à la fois la contention médicale recherchée définie plus haut et un port plus frais, plus aéré qu'avec les mailles des bas ou collants de contention classiques.

Cette partie de jambe est, de façon caractéristique de l'invention, réalisée en tricotant une maille avec motif résille, par exemple par charge de maille et tricotage double serre progressive.

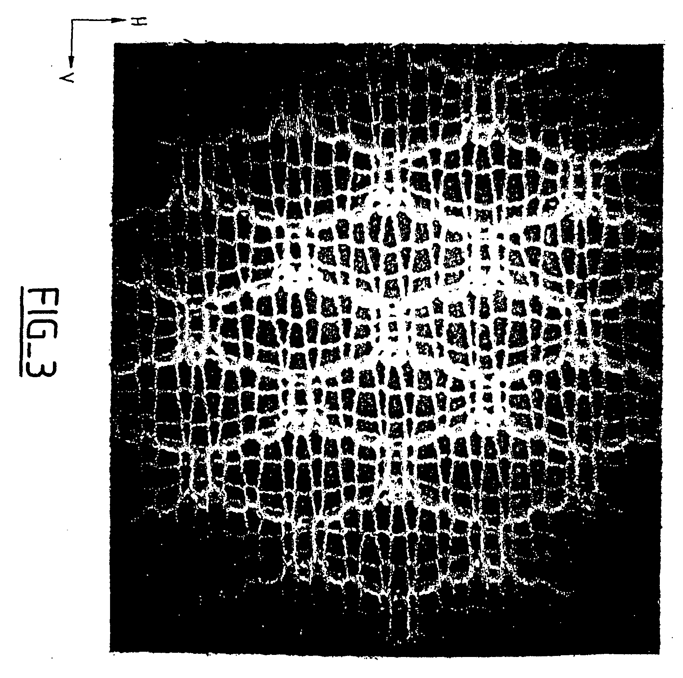

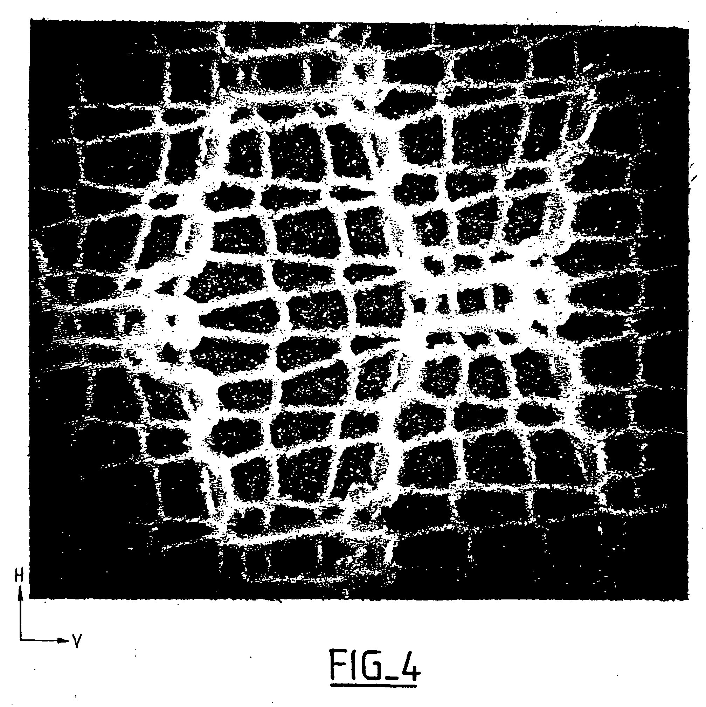

Ce tricotage utilise essentiellement deux types de fils, illustrés plus en détail sur la figure 2 et visibles sur les macrophotographies des figures 3 et 4, à savoir :
― un fil de trame 22, par exemple un fil d'élasthanne 130 dtex double guipé polyamide 28 dtex à 28 filaments plats, et
― un ou plusieurs fils de fond 24, 26, 28, typiquement des fils élasthanne 33 dtex double guipé 8 dtex à 5 filaments plats (titrage en soi typique d'un bas conventionnel, non thérapeutique).

Le choix de matières indiqué ci-dessus (élasthanne guipé polyamide, avec une composition 31 % élasthanne + 69 % polyamide) n'est pas limitatif, d'autres compositions ou proportions pouvant être utilisées, par exemple un élasthanne guipé coton et polyamide, ou encore un mélange d'élasthanne et d'élasto-diène (latex de caoutchouc).

Il est également possible d'utiliser pour les fils de fond, ou pour les fils de trame, ou encore pour le guipage des fils de fond ou de trame, des fibres thermorégulatrices telles que le Meryl® de Nylstar (fil polyamide creux) ou le Tactel® de du Pont de Nemours (fil polyamide profilé), ou encore une fibre contenant des microcapsules à changement de phase ou des microcapsules rafraîchissantes.

Le tricotage de la maille de l'invention peut être réalisé sur une machine à tricoter programmable à cylindre 4 pouces (10 cm) à 328 aiguilles, avec une rangée sur quatre constituée par le fil de trame 130 dtex, et trois rangées sur quatre par les fils de fond 33 dtex.

Le tricotage est réalisé de manière à former avec le fil de trame un réseau de cellules sensiblement hexagonales 30. Les rangées successives de fil de trame sont nouées entre elles en 32 (ce nouage n'a pas été représenté sur la figure 2 pour la clarté de la figure, mais il est visible sur les photographies des figures 3 et 4). Les deux parties de fil 34 s'étendant de part et d'autre de ce nouage 32 sont resserrées en 36 par l'un des fils de fond 24 pour former l'un des côtés de l'hexagone en rapprochant les deux parties 34 l'une de l'autre.

Le nouage 32 permet en particulier d'obtenir une contention suffisante, le caractère extensible de la maille résultant essentiellement de l'élasticité du fil de trame plus que du tricotage particulier de la maille.

Les fils de fond 24, 26, 28 forment en outre un maillage remplissant chacune des cellules 30 pour constituer un fin réseau donnant une texture au bas sans pour autant empêcher la déformation élastique de celui-ci qui permet d'obtenir la contention recherchée.

Sur les figures, les directions V et H indiquent respectivement l'orientation de la verticale et de l'horizontale par rapport à la jambe lorsque le patient est debout. Ainsi, les fils de trame 22 et de fond 24, 26 et 28 suivent tous la direction horizontale H (celle du fil de trame), car c'est la déformation élastique dans cette direction qui procurera la contention recherchée.

La dimension des cellules 30 sera choisie en fonction du degré de contention recherché, un quadrillage plus large procurant une contention moindre, pour un même choix de fils, qu'un quadrillage serré.

Dans une variante, pour obtenir une pression de contention plus élevée, on peut choisir pour l'un des fils de fond 24, 26 et/ou 28 un fil non élastique formant fil de frein.

L'effet thérapeutique recherché (pression efficace et dégressivité depuis la cheville) étant conservé en totalité, les indications thérapeutiques du bas ou du collant de contention selon l'invention seront identiques à celles des bas ou collants classiques, typiquement pour des classes I ou II.

## Revendications

1. Une orthèse compressive du type bas ou collant de contention comprenant une partie de jambe (16) en maille compressive apte à délivrer une contention thérapeutique dégressive, orthèse **caractérisée en ce que** ladite maille compressive comprend une maille de type résille tricotée à partir de rangées successives de fil de trame (22) en matière élastique nouées entre elles de manière à former un réseau de cellules (3), et d'un maillage de fils de fond (24, 26, 28) tricotés de manière à remplir lesdites cellules (3).

2. L'orthèse compressive de la revendication 1, dans laquelle la maille de type résille comprend au moins un fil de fond (24, 26, 28) en matière élastique, de plus faible titrage que le fil de trame.

3. L'orthèse compressive de la revendication 2, dans laquelle la maille comprend au moins deux fils de fond pour un fil de trame, de préférence trois fils de fond (24, 26, 28) pour un fil de trame (22).

4. L'orthèse compressive de la revendication 3, dans laquelle le(s) fil(s) de fond est (sont) tricoté(s) avec le fil de trame de manière à former avec celui-ci un réseau déformable de cellules sensiblement hexagonales (30).

5. L'orthèse compressive de la revendication 4, dans laquelle le réseau déformable présente à la déformation une plus grande force de rappel élastique dans la direction horizontale (H) que dans la direction verticale (V), la direction horizontale étant celle du fil de trame (22).

6. L'orthèse compressive de la revendication 2, dans laquelle le ratio du titrage du fil de fond par rapport au titrage du fil de trame est d'au moins 3:1, de préférence environ 4:1.

7. L'orthèse compressive de la revendication 6, dans laquelle le titrage du fil de trame est d'environ 130 dtex, pour un titrage du fil de fond d'environ 33 dtex.

## Claims

1. A compressive orthosis of the elastic stocking or tights type, having a leg portion (16) in a compressive knit suitable for delivering degressive therapeutic retention, the orthosis being **characterized in that** said compressive knit comprises a net type knit knitted from successive rows of weft thread (22) of elastic material mutually knotted to form an array of cells (3), and a mesh of background threads (24, 26, 28) knitted so as to fill said cells (30).

2. The compressive orthosis of claim 1, in which the net type knit includes at least one background thread (24, 26, 28) of elastic material, and of smaller weight than the weft thread.

3. The compressive orthosis of claim 2, in which the knit comprises at least two background threads for each weft thread, and preferably three background threads (24, 26, 28) for each weft thread (22).

4. The compressive orthosis of claim 3, in which the background thread(s) is(are) knitted with the weft thread in such a manner as to co-operate therewith to form a deformable array of substantially hexagonal cells (30).

5. The compressive orthosis of claim 4, in which the deformable array, on being deformed, presents a greater resilient return force in the horizontal direction (H) than in the vertical direction (V), said horizontal direction being the direction of the weft thread (22).

6. The compressive orthosis of claim 2, in which the weight ratio of the background thread to the weft thread is at least 1:3 and preferably about 1:4.

7. The compressive orthosis claim 6, in which the weight of the weft thread is about 130 dtex and that of the background thread is about 33 dtex.

## Patentansprüche

1. Eine kompressive Orthese vom Typ eines Strumpfs oder einer Strumpfhose zum Stützen, aufweisend eine Beinpartie (16) aus kompressiven Maschen, dazu geeignet, eine degressive therapeutische Stützung zu liefern, wobei die Orthese **dadurch gekennzeichnet ist, dass** die kompressiven Maschen Maschen vom Typ eines gestrickten Gitters umfassen, ausgehend von aufeinander folgenden Reihen von Schussfäden (22) aus elastischem Material, welche zwischen jenen geknüpft sind, derart, um ein Gitter von Zellen (3) zu formen, und eines Netzes von Grundfäden (24, 26, 28), derart gestrickt, um die Zellen (3) zu füllen.

2. Kompressive Orthese nach Anspruch 1, worin die Masche vom Stricktyp wenigstens einen Grundfaden (24, 26, 28) aus elastischem Material umfasst, welcher eine schwächere Titrierung als der Schussfaden hat.

3. Kompressive Orthese nach Anspruch 2, worin die Masche wenigstens zwei Grundfäden für einen Schussfaden, vorzugsweise drei Grundfäden (24, 26, 28) für einen Schussfaden (22) umfasst.

4. Kompressive Orthese nach Anspruch 3, worin der (die) Grundfaden (-fäden) mit dem Schussfaden derart gestrickt ist (sind), um mit diesem ein deformierbares Gitter von im Wesentlichen hexagonalen Zellen (30) zu bilden.

5. Kompressive Orthese nach Anspruch 4, worin das deformierbare Gitter bei der Deformation eine größere elastische Rückzugskraft in der horizontalen Richtung (H) als in der vertikalen Richtung (V) aufweist, wobei die horizontale Richtung die des Schussfadens (22) ist.

6. Kompressive Orthese nach Anspruch 2, worin das Verhältnis der Titrierung des Grundfadens im Verhältnis zur Titrierung des Schussfadens wenigstens 3:1, vorzugsweise ungefähr 4:1 ist.

7. Kompressive Orthese nach Anspruch 6, worin die Titrierung des Schussfadens ungefähr 130 dtex ist, für eine Titrierung des Grundfadens von ungefähr 33 dtex.
